# EUROPEAN PATENT APPLICATION

(11) **EP 2 412 292 A1**
(43) Date of publication of application: **01.02.2012**
(21) Application number: 10755891.8
(22) Date of filing: 12.03.2010
(51) Int. Cl.: A61B 1/00, A61B 5/07

(54) **CAPSULE MEDICAL DEVICE AND CAPSULE MEDICAL SYSTEM**

(30) Priority: 24.03.2009 JP 2009072847
(71) Applicant: Olympus Corporation, Shibuya-ku Tokyo 151-0072 (JP)
(72) Inventor: ISHIHARA, Yasushige, Tokyo 151-0072 (JP)
(74) Representative: Gunzelmann, Rainer
(86) International application number: PCT/JP2010/054217
(87) International publication number: WO 2010/110091

(57) **Abstract**

The burden on a patient is reduced, and acquired measurement information is readily and reliably collected. A capsule-type medical device (10) is provided, including a capsule-type casing (12) that is introduced into a body cavity of a patient, a measurement-information acquisition unit (20) that is disposed inside the casing and that acquires measurement information about a site to be examined in the body cavity; an IC chip (32) that stores the measurement information acquired by the measurement-information acquisition unit (20); a capsule antenna (34) that receives from outside the body cavity a measurement-information-reading signal and that transmits outside the body cavity the measurement information stored in the IC chip (32); a pH sensor unit (40) that determines a passing position by acquiring positional information in the body cavity; and a buzzer (50) that issues a notification sound outside the body cavity when the passing position determined by the pH sensor unit (40) satisfies a predetermined positional condition.

## Description

### {Technical Field}

The present invention relates to a capsule-type medical device and a capsule-type medical system including the same.

### {Background Art}

In the related art, there is a capsule-type medical device that has a capsule-type casing that is introduced into a patient's body and that acquires image information by capturing images of the inside of a body cavity (for example, see Patent Literatures 1, 2, and 3). The capsule-type medical device disclosed in Patent Literature 1 temporarily saves acquired image information with image saving means in a capsule-type casing and successively transmits the image information via wireless transmission means to an external device disposed outside the patient's body. However, with the successive transmission of the image information to the external device via the wireless transmission means, examination must be performed while the patient is wearing a portable external device or remains close to a stationary external device, and there is a problem in that the patient feels restrained and subjected to a large burden. In addition, although it is conceivable that the image information saved in the image saving means is collected in an external storage device or the like after the capsule-type casing is excreted from the patent's body, there is a risk of losing the capsule-type casing at the time of excretion, making it impossible to collect the image information saved in the casing.

In contrast, a capsule-type medical device disclosed in Patent Literature 2 detects the excretion of a capsule-type casing outside the body with detection means and externally reports the fact with notification means. In addition, a capsule-type medical device disclosed in Patent Literature 3 recognizes that a capsule-type casing is located in the large intestine with recognition means and reports that the capsule-type casing is about to be excreted by outputting a notification signal outside the body with notification means.

### {Citation List}

### {Patent Literature}

{PTL 1} Japanese Unexamined Patent Application, Publication No. 2003-70728.
{PTL 2} Japanese Unexamined Patent Application, Publication No. 2004-261522.
{PTL 3} Japanese Unexamined Patent Application, Publication No. 2004-329749.

### {Summary of Invention}

### {Technical Problem}

However, even if it is possible to prevent a capsule-type casing from being lost by reporting that the capsule-type casing has been excreted from the patent's body or that the capsule-type casing is about to be excreted, as with the capsule-type medical devices disclosed in Patent Literatures 1 and 2, the capsule-type casing and image information therein must be recovered from stool excreted together with the capsule-type casing, and there is a problem in that doing so is time-consuming and unsanitary.

The present invention has been conceived in light of such circumstances, and an object thereof is to provide a capsule-type medical device that is capable of reducing the burden on a patent and that also allows acquired measurement information to be readily and reliably collected and to provide a capsule-type medical system including the same.

### {Solution to Problem}

The present invention employs the following solutions in order to solve the above-described problems.
A first aspect of the present invention provides a capsule-type medical device including a capsule-type casing that is introduced into a body cavity of a patient; a measurement-information acquisition unit that is disposed inside the casing and that acquires measurement information about an examination-target site in the body cavity; a storage unit that stores the measurement information acquired by the measurement-information acquisition unit; a transmitting/receiving unit that receives from outside the body cavity a measurement-information-reading signal and that transmits outside the body cavity the measurement information stored in the storage unit; a passing-position determining unit that determines passing position by acquiring positional information in the body cavity; and a notification unit that reports the passing information outside the body cavity when the passing position determined by the passing-position determining unit satisfies a predetermined positional condition.

With this aspect, when the capsule-type casing is introduced into the body cavity of the patient, the measurement information of the examination-target site in the body cavity is acquired through the operation of the measurement-information acquisition unit and is stored in the storage unit, and the positional information in the body cavity is also acquired by the operation of the passing-position determining unit, and the passing position is determined. Then, when the measurement-information-reading signal is sent from outside the body cavity, the measurement information stored in the storage unit is transmitted outside the body cavity by the operation of the transmitting/receiving unit.

With this capsule-type medical device, when the passing position determined by the passing-position determining unit satisfies the predetermined positional condition, the passing information is reported outside the body cavity by the operation of the notification unit; therefore, the patient, a doctor, etc. who have recognized the passing information from the notification unit send the measured-information-reading signal to the transmitting/receiving unit, thereby making it possible to read out the measured information from a desired examination-target site before the capsule-type medical device is excreted.

Therefore, the measured information from the desired examination-target site can be collected at an appropriate time without having the patient wear an external device or stay close to an external device. Accordingly, the burden on the patient can be reduced, and the acquired measurement information can be readily and reliably collected. The measurement information includes, for example, fluorescence intensity acquired by detecting fluorescence generated at the site to be examined and fluorescence image information acquired by capturing the fluorescence.

In the above-described aspect, the measurement-information acquisition unit may include a light source that radiates excitation light onto the examination-target site, a fluorescence selecting unit that selectively transmits fluorescence of a predetermined band generated at the examination-target site by the excitation light irradiated from the light source, and a fluorescence capturing unit that acquires fluorescence image information by capturing the fluorescence that has passed through the fluorescence selecting unit.

By employing such a configuration, the diagnostic capability can be enhanced by acquiring fluorescence image information having greater sensitivity and specificity for lesions such as cancer cells, etc. The fluorescence image information may be a fluorescence image in which agent fluorescence generated at an examination-target site is captured by supplying, in advance, a fluorescent agent that accumulates specifically in a lesion in the body cavity or it may be a fluorescence image in which autofluorescence generated at an examination-target site is captured.

In the above-described aspect, the measurement-information acquisition unit may include a light source that radiates excitation light onto the examination-target site and a light detector that detects intensity information of fluorescence of a predetermined band generated at the examination-target site by the excitation light irradiated from the light source.
By detecting the fluorescence intensity information with the light detector, the amount of information to be stored in the storage unit can be reduced as compared with storing the image information, which can facilitate examination over an extended period of time.

In the above-described aspect, the storage unit may store the measurement information acquired by the measurement-information acquisition unit in association with the passing position determined by the passing-position determining unit.
By employing such a configuration, the correspondence between the collected measurement information and positions of examination-target sites in the body can be readily checked after the examination.

In the above-described aspect, a read-out restricting unit that restricts the transmitting/receiving unit from transmitting the measurement information outside the body cavity until the passing position determined by the passing-position determining unit satisfies a predetermined positional condition may be provided
By employing such a configuration, with the read-out restricting unit, it is possible to prevent image information with an insufficient amount of information from being accidentally read out before examination of a desired site is completed.

In the above-described aspect, the passing-position determining unit may be a timer that measures elapsed time.
By employing such a configuration, the passing position in the body cavity can be determined based on the elapsed time since introduction of the capsule-type medical device into the body cavity.

In the above-described aspect, the passing-position determining unit may be a pressure sensor that detects pressure exerted on the casing.
By employing such a configuration, the passing position in the body cavity can be determined based on the pressure exerted on the casing and detected by the pressure sensor. For example, it can be judged that the passing position is in relatively narrow organ, such as the esophagus, the small intestine, the large intestine, etc. when the pressure received by the casing is high, and that the passing position is in relatively large organ, such as the stomach, when the pressure is low.

In the above-described aspect, the passing-position determining unit may be an intestinal-bacteria sensor that detects intestinal bacteria.
By employing such a configuration, when the intestinal bacteria are detected by the intestinal-bacteria sensor, it can be determined in a simple manner that the capsule-type medical device has moved into the large intestine.

In the above-described aspect, the passing-position determining unit may be a pH sensor that detects a pH value in the area around the casing.
Because the pH value is low in the stomach as compared with other organs, by detecting the pH value in the area around the casing with the pH sensor, it can be determined in a simple manner that the capsule-type medical device has moved into the stomach based on the output of the pH sensor.

In the above-described aspect, the passing-position determining unit may be a fluorescence detecting sensor that detects fluorescence in the area around the casing.
By employing such a configuration, ICG (indocyanine green) is supplied to the body cavity in advance, stool dyed by mixing with ICG excreted from bile is detected with the fluorescence detecting sensor, and thus it is possible to determine in a simple manner that the capsule-type medical device has moved into the large intestine.

In the above-described aspect, the passing-position determining unit may be a gas sensor that detects gas in the area around the casing.
The large intestine contains methane gas emitted from the stool, and the concentration of the methane gas is particularly high near the rectum where stool is accumulated; therefore, by detecting the gas in the area around the casing with the gas sensor, it can be determined in a simple manner that the capsule-type medical device has moved into the large intestine based on the presence of the methane gas. In addition, based on the amount of emitted methane gas, it can be judged whether or not the capsule-type medical device is about to be excreted.

In the above-described aspect, the passing-position determining unit may be a color detecting sensor that detects color in the area around the casing.
By employing such a configuration, it can be judged that the capsule-type medical device is about to be excreted if a state in which the color of stool is detected by the color detecting sensor continues for a certain amount of time or longer.

A first aspect of the present invention provides a capsule-type medical system including a capsule-type medical device according the above-described first aspect; a measurement-information reading unit that transmits the measurement-information-reading signal and that reads the measurement information sent from the transmitting/receiving unit; and a measurement-information saving unit that saves the read measurement information.
With this aspect, the burden on the patent can be reduced, and the measurement information from a desired examination-target site can be saved by readily and reliably collecting it at an appropriate time.

### {Advantageous Effects of Invention}

With the present invention, an advantage is afforded in that the burden on a patient can be reduced, and acquired measurement information can be readily and reliably collected.

### {Brief Description of Drawings}

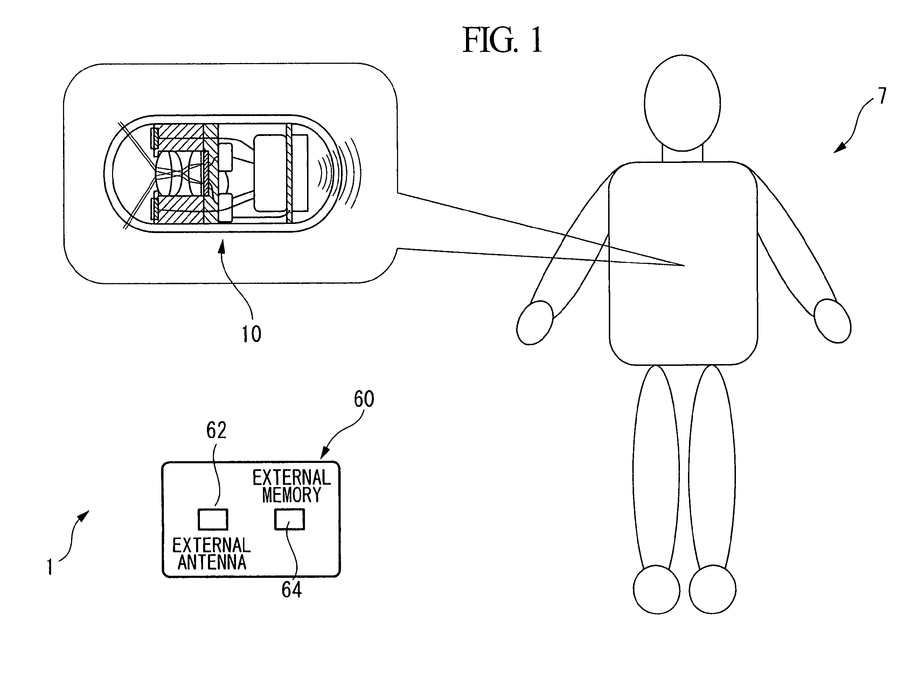

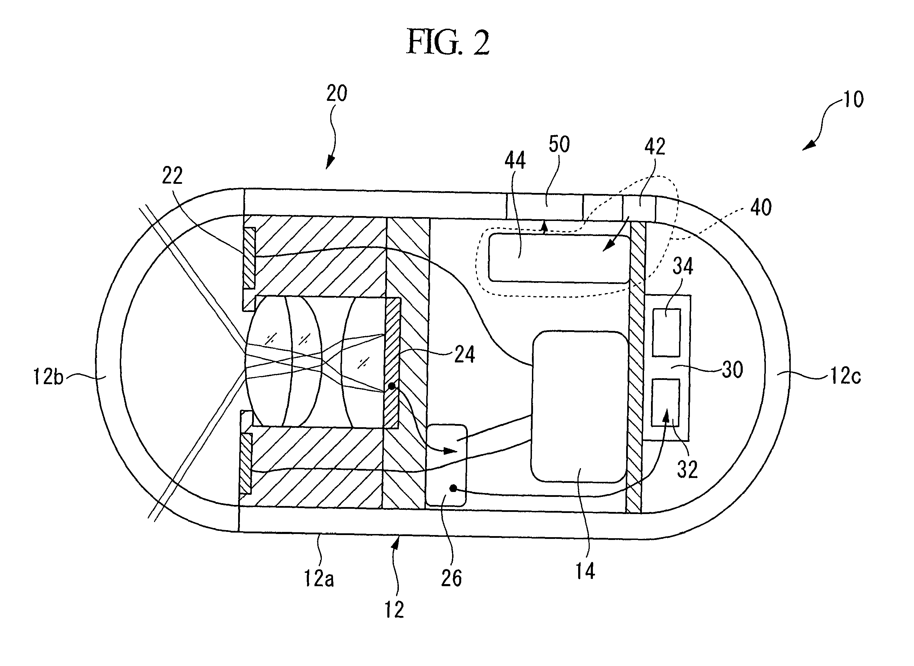

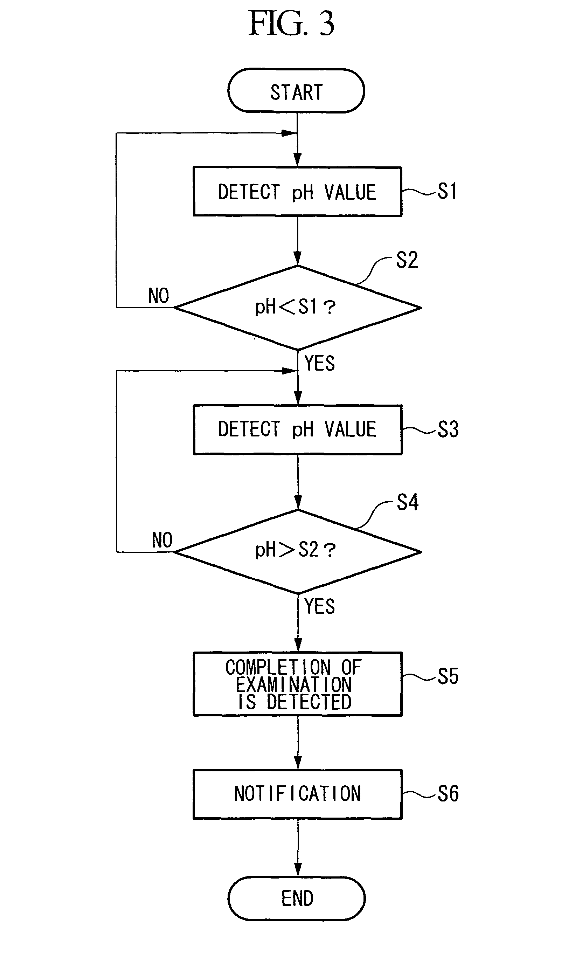

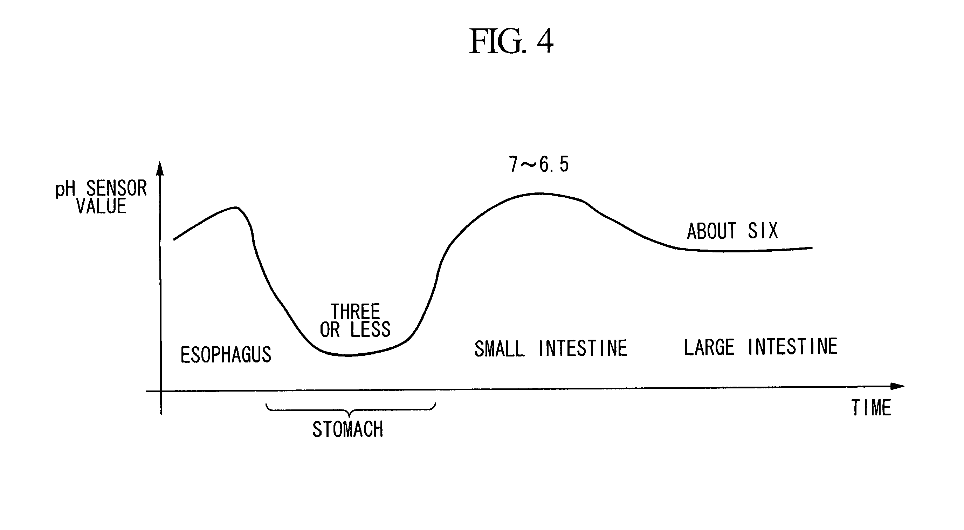

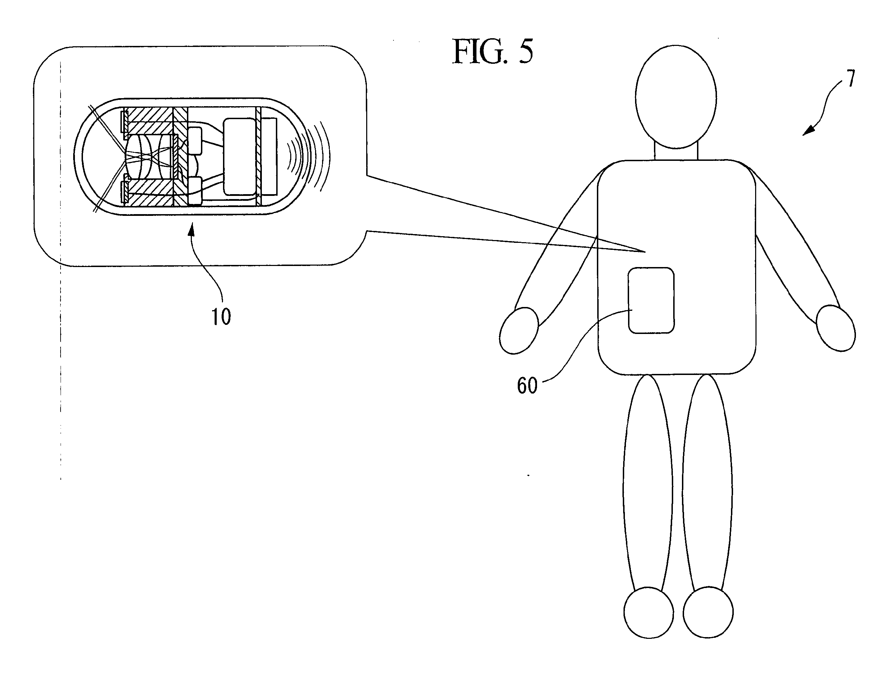

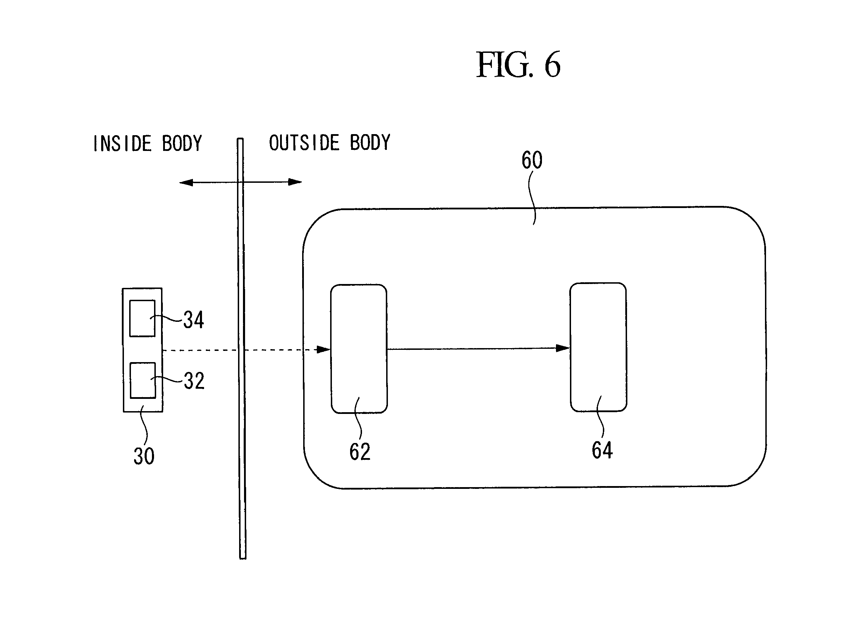

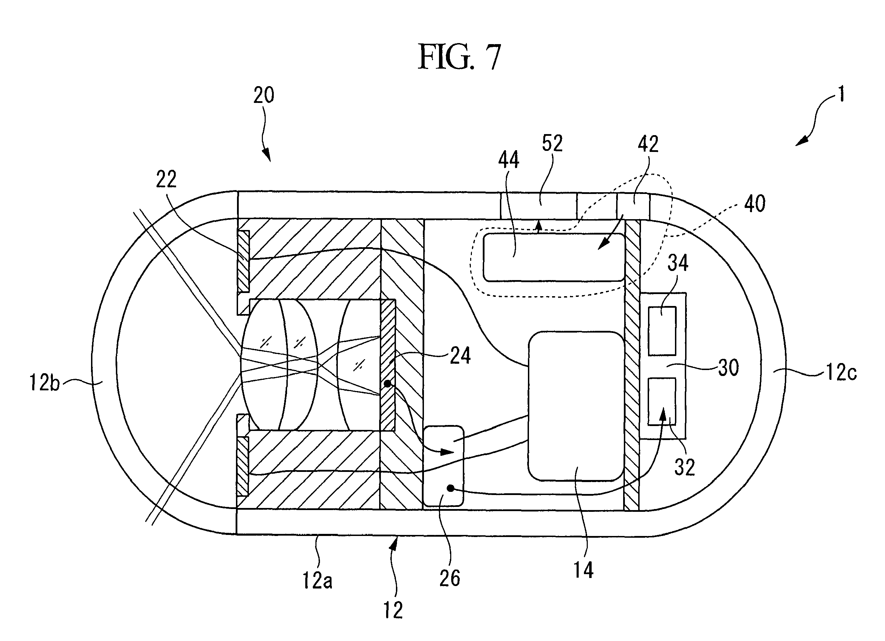

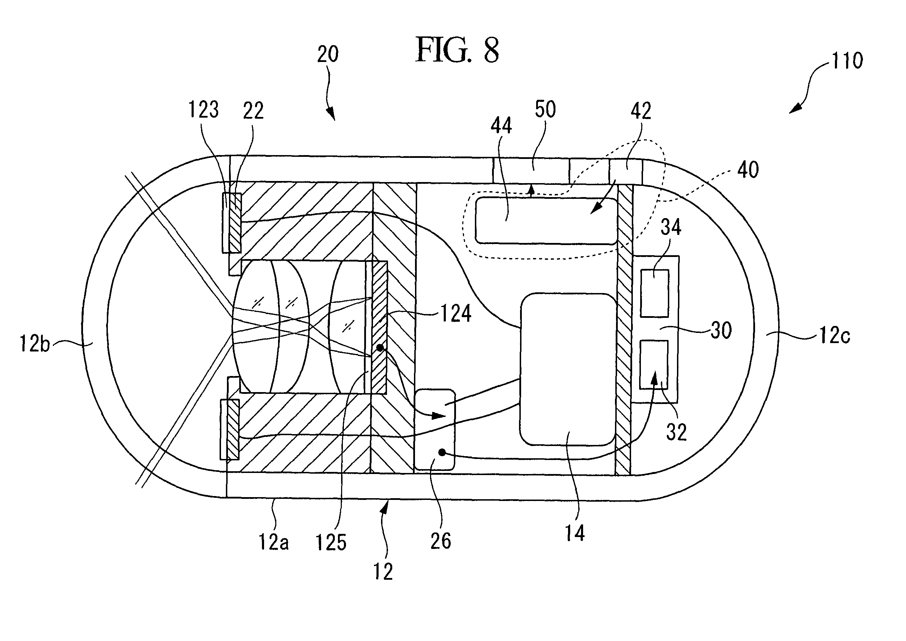

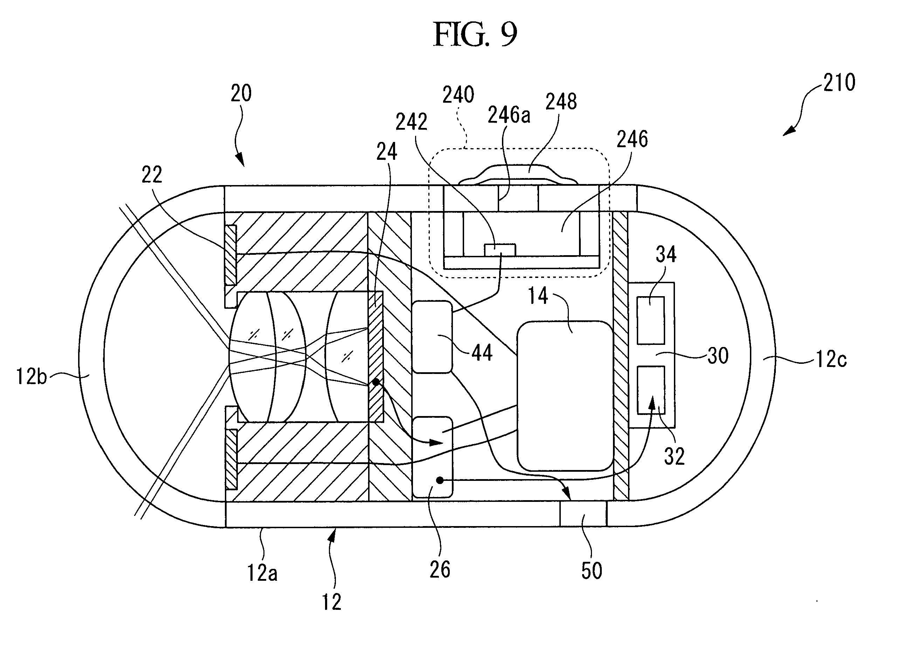

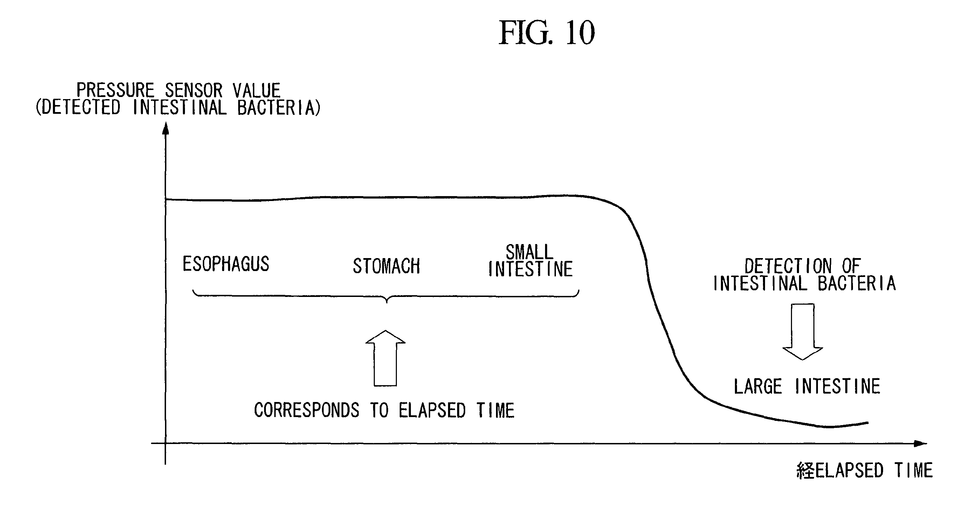

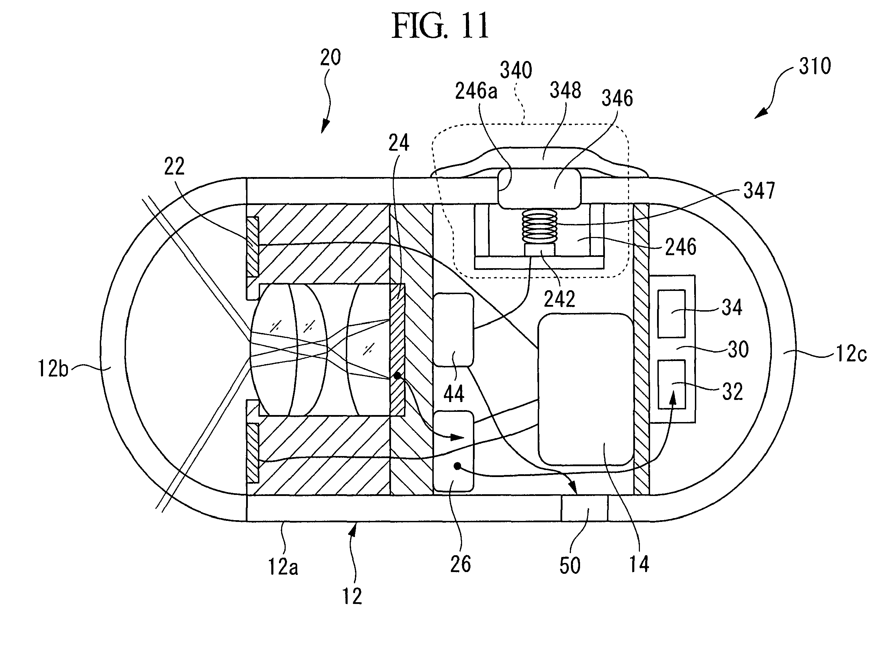

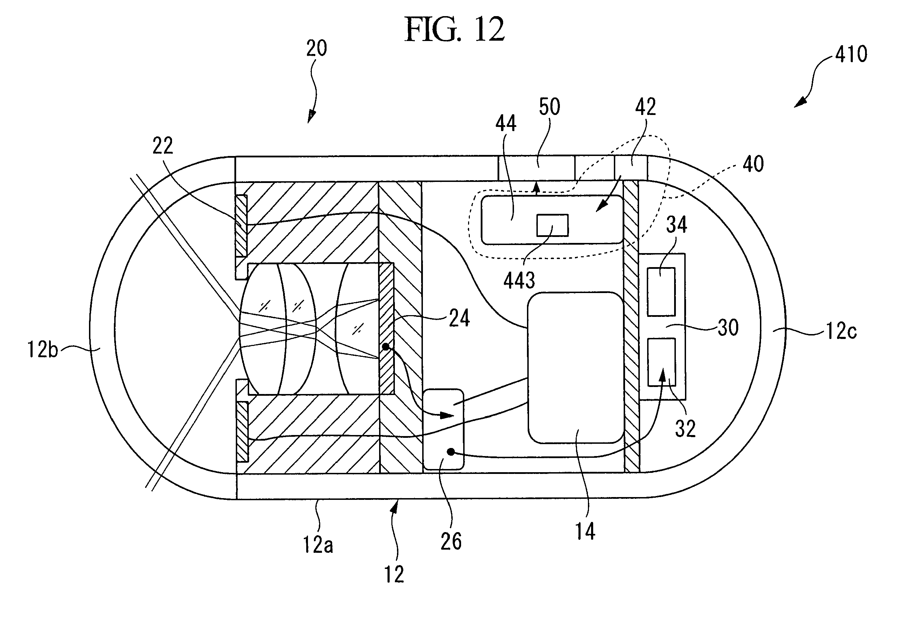

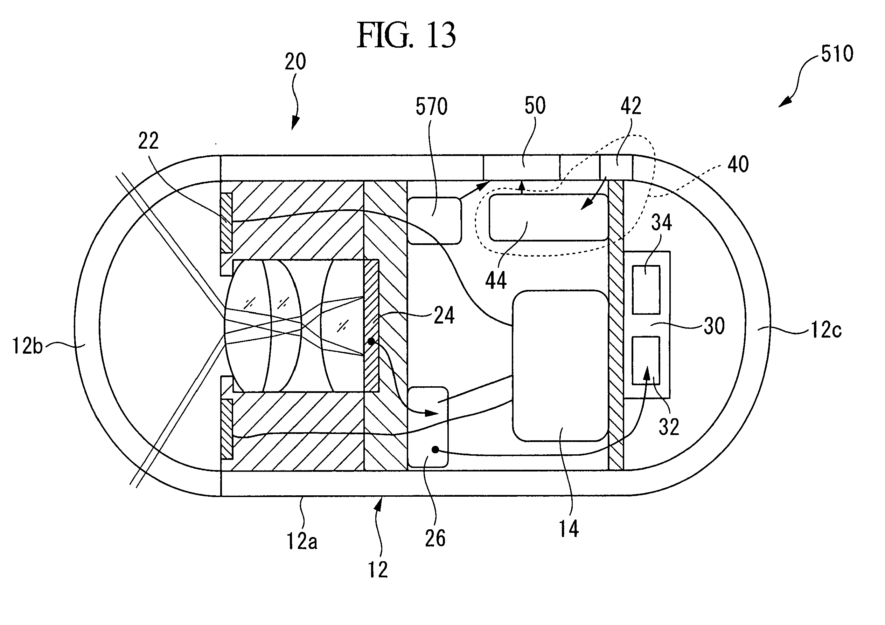

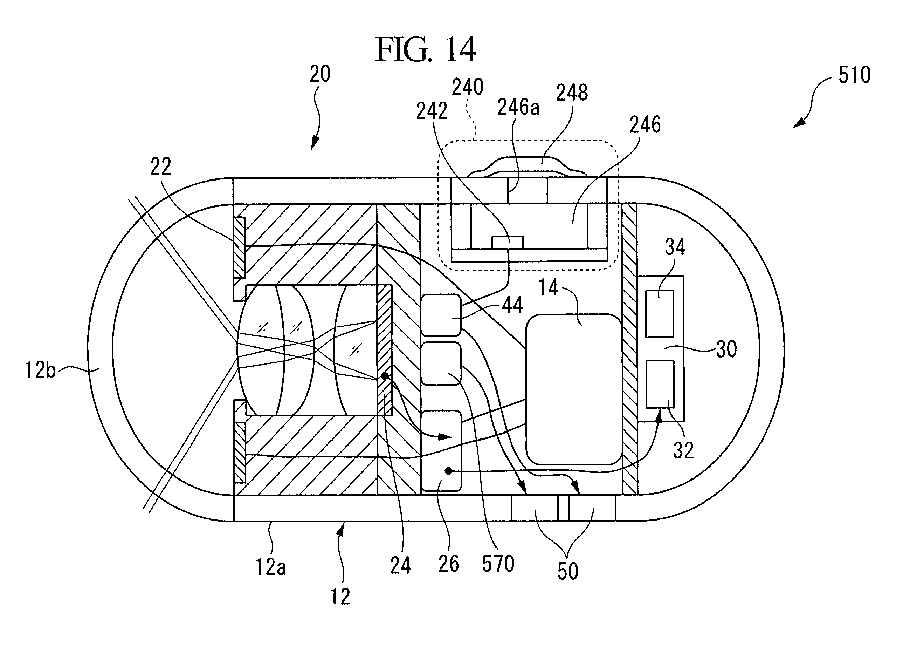

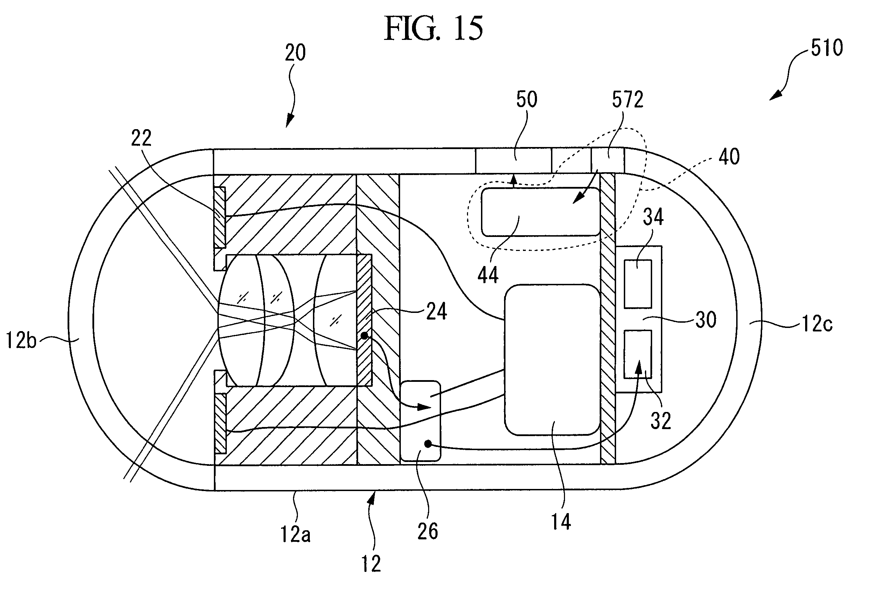

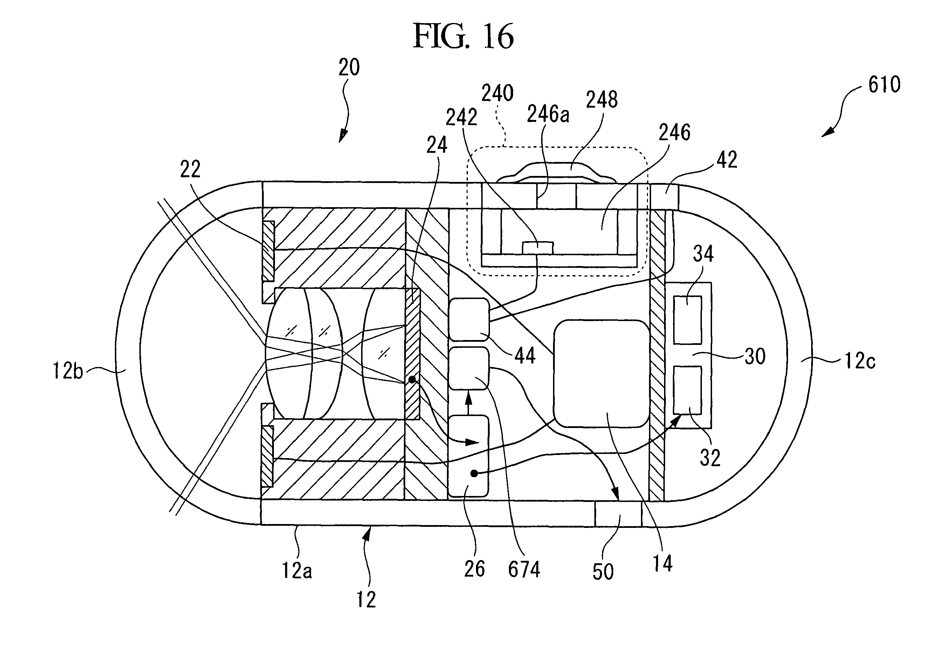

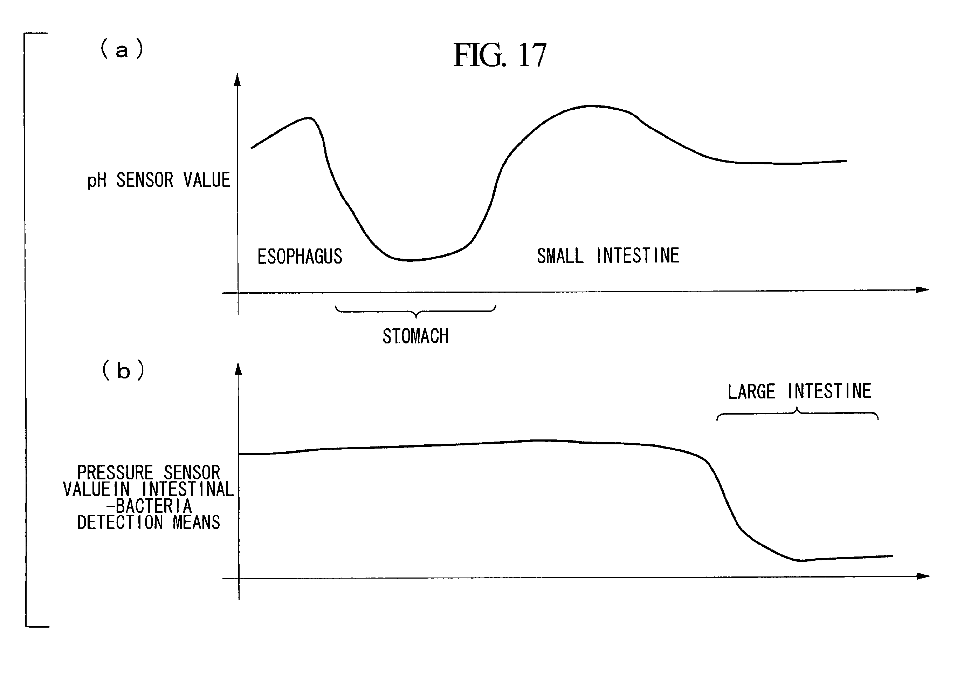

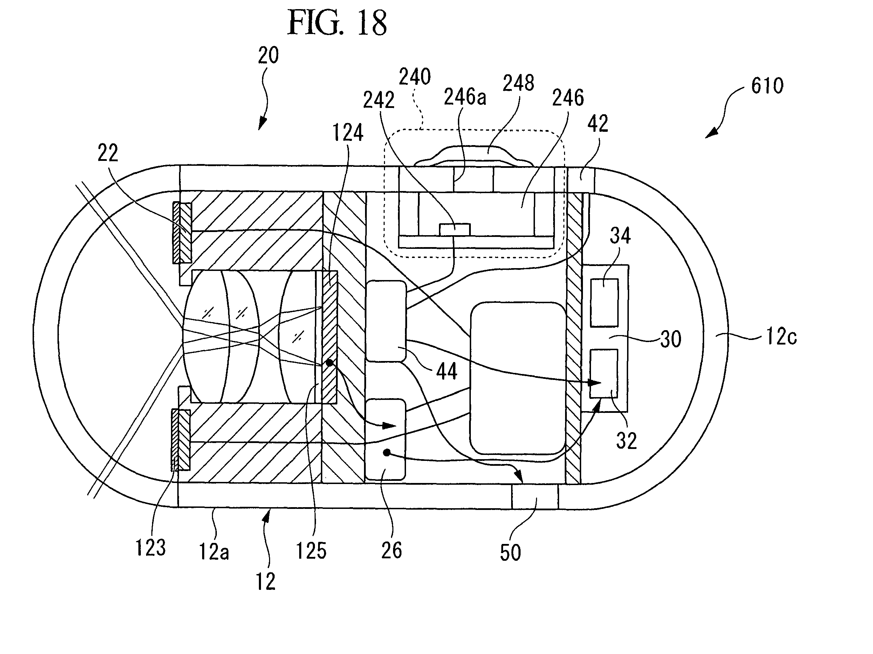

{Fig. 1} Fig. 1 is a schematic view showing a capsule-type medical system according to a first embodiment of the present invention.
{Fig. 2} Fig. 2 is a schematic view showing a capsule-type medical device in Fig. 1.
{Fig. 3} Fig. 3 is a flowchart showing the procedure of determining a passing position of a pH sensor unit in Fig. 1.
{Fig. 4} Fig. 4 is a diagram showing changes in pH value detected by the pH sensor in Fig. 1.
{Fig. 5} Fig. 5 is a diagram showing a manner in which image information is collected from the capsule-type medical device in a body cavity by using an external device.
{Fig. 6} Fig. 6 is a diagram showing a manner in which the image information is read out from the capsule-type medical device in a body cavity by using an external device.
{Fig. 7} Fig. 7 is a schematic view showing a capsule-type medical device according to a modification of the first embodiment of the present invention.
{Fig. 8} Fig. 8 is a schematic view showing a capsule-type medical device according to a first modification of the first embodiment of the present invention.
{Fig. 9} Fig. 9 is a schematic view showing a capsule-type medical device according to a second modification of the first embodiment of the present invention.
{Fig. 10} Fig. 10 is a diagram showing changes in pressure detected by an intestinal bacteria sensor unit of the capsule-type medical device according to the second modification of the first embodiment of the present invention.
{Fig. 11} Fig. 11 is a schematic view showing a capsule-type medical device according to a third modification of the first embodiment of the present invention.
{Fig. 12} Fig. 12 is a schematic view showing a capsule-type medical device according to a fourth modification of the first embodiment of the present invention.
{Fig. 13} Fig. 13 is a schematic view showing a capsule-type medical device according to a second embodiment of the present invention.
{Fig. 14} Fig. 14 is a schematic view showing a capsule-type medical device according to a modification of the second embodiment of the present invention.
{Fig. 15} Fig. 1 is a schematic view showing a capsule-type medical device according to another modification of the second embodiment of the present invention.
{Fig. 16} Fig. 16 is a schematic view showing a capsule-type medical device according to a third embodiment of the present invention.
{Fig. 17} Fig. 17(a) is a diagram showing changes in pH value detected by a pH sensor in Fig. 16, and Fig. 17(b) is a diagram showing changes in pressure detected by an intestinal bacteria sensor unit in Fig. 16.
{Fig. 18} Fig. 18 is a schematic view showing a capsule-type medical device according to a modification of the third embodiment of the present invention.

### {Description of Embodiments}

### {First Embodiment}

A capsule-type medical device, and a capsule-type medical system including the same, according to a first embodiment of the present invention will be described below with reference to the drawings.
As shown in Fig. 1, a capsule-type medical system 1 according to this embodiment is provided with a capsule-type medical device 10 that is introduced into a body cavity of a patient 7 and that acquires image information (measurement information) of a site to be examined and an external device (measurement-information collection device) 60 disposed outside the body cavity that collects the image information acquired by the capsule-type medical device 10.

As shown in Fig. 2, the capsule-type medical device 10 is provided with a capsule-type casing 12 in which two ends of a cylindrical casing main body 12a are sealed with a semispherical transparent window 12b an a end plate 12c. The casing 12 internally is provided with an image-information acquisition unit (measurement-information acquisition unit) 20 that irradiates the site to be examined outside the casing 12 with illumination light via the transparent window 12b and that acquires image information by capturing returning light from the site to be examined, which enters the casing 12 via the transparent window 12b; an information managing unit 30 that manages the image information acquired by the image-information acquisition unit 20; a pH sensor unit (passing-position determining unit) 40 that determines passing positions by acquiring positional information in the body cavity; and a buzzer (notification unit) 50 that issues a notification sound (passing information) outside the body cavity on the basis of a notification signal input from the pH sensor unit 40. Reference sign 14 is a battery that supplies power to these various parts.

The image-information acquisition unit 20 is provided with an LED (light source) 22 that emits the illumination light, a CCD (or CMOS) 24 that acquires image information by capturing the returning light, and an image generating unit 26 that reads the image information acquired by the CCD 24 and generates a two-dimensional image.

The information managing unit 30 is provided with an IC chip (storage unit) 32 that stores the image information sent from the image generating unit 26, and a capsule antenna (transmitter/receiver unit) 34 that transmits the image information stored in the IC chip 32 outside the body cavity. The capsule antenna 34 is configured so as to output the image information by receiving an image-information-reading signal (measurement-information-reading signal) from outside the body cavity.

The pH sensor unit 40 is provided with a pH sensor 42 that detects the pH value (positional information) of the area around the casing 12 and a sensor control unit 44 that determines passing position on the basis of the pH value detected by the pH sensor 42 and judges whether or not the determined passing position satisfies a predetermined positional condition.

The sensor control unit 44 is configured so that a threshold for the pH value can be set therein. The sensor controller 44 determines the passing position by comparing a pH value detected by the pH sensor 42 and a preset threshold and outputs a notification signal to the buzzer 50 when the determined passing position satisfies the predetermined positional condition.

The external device 60 is provided with an external antenna (measurement-information-reading unit) 62 that transmits the image-information-reading signal and reads the image information sent from the capsule antenna 34 of the capsule-type medical device 10 disposed inside the body cavity and an external memory (measurement-information saving unit) 64 that saves the image information read by the external antenna 62.

The operations of the thus-configured capsule-type medical device 10, and the capsule-type medical system 1 including the same, according to this embodiment will be described.
To examine, for example, the stomach of the patient 7 by employing the capsule-type medical device 10 according to this embodiment, first the threshold for the pH value and the positional condition are input to the sensor control unit 44, and the capsule-type medical device 10 is orally introduced. Inside the body cavity, the illumination light is radiated onto the site to be examined via the transparent window 12b by activating the LED 22, and the returning light returning from the site to be examined enters the casing 12 via the transparent window 12b.

The returning light that has entered the casing 12 is captured by the CCD 24 and is acquired as image information. The image information acquired by the CCD 24 is input to the image generating unit 26 where a two-dimensional image is generated, and the image information is also input to the information managing unit 30 to be stored in the IC chip 32.

As shown in the flowchart in Fig. 3, with the capsule-type medical device 10 according to this embodiment, the pH sensor 42 of the pH sensor unit 40 detects a pH value in the area around the casing 12, and the sensor control unit 44 compares the pH value detected by the pH sensor 42 with the preset threshold and determines a passing position.

Generally, gastric juice has a pH of about 2 to 3. When the capsule-type medical device 10 passes through the esophagus and reaches the stomach, the pH changes from neutral pH to strongly acidic pH. That is, as shown in Fig. 4, whereas the value is 6 or higher while passing through the esophagus, the pH value detected by the pH sensor 42 drops to 3 or lower upon reaching the stomach. Therefore, a threshold S1 is set at, for example, a pH value of 5. In Fig. 4, the longitudinal axis indicates the pH value detected by the pH sensor 42, and the horizontal axis indicates the elapsed time since the capsule-type medical device was introduced into the patient 7.

In addition, because the pH value is no longer lowered by the gastric juice in parts that follow the duodenum on the downstream side of the stomach, once the capsule-type medical device 10 moves to the small intestine and the large intestine, pH value detected by the pH sensor 42 increases to 6 or higher as shown in Fig. 4. Therefore, a threshold S2 is set at, for example, a pH value of 6. In addition, the positional condition is set at a point where the pH value exceeds the threshold S2 again.

Until the capsule-type medical device 10 reaches the stomach, pH value detected by the pH sensor 42 is above the threshold S1 ("NO" in Step 1 and Step 2). When the capsule-type medical device 10 reaches the stomach and the pH sensor 42 detects the pH value therein (Step 1), the pH value is lower than the threshold S1 ("YES" in Step 2). Accordingly, the sensor control unit 44 judges that the capsule-type medical device 10 is passing through the stomach.

Next, after the capsule-type medical device 10 passes through the stomach and until it reaches the duodenum, the pH value detected by the pH sensor 42 is lower than the threshold S2 ("NO" in Step 3 and Step 4). When the capsule-type medical device 10 moves to the duodenum and the pH sensor 42 detects the pH value therein (Step 3), the pH value is above the threshold S2 ("YES" in Step 4). Accordingly, the sensor control unit 44 judges that the capsule-type medical device 10 is passing through the duodenum.

Then, at the sensor control unit 44, it is judged that the pH value exceeds the threshold S2 again, and that the predetermined positional condition is satisfied, that is, that the capsule-type medical device 10 has moved to the duodenum and the examination of the stomach has been completed (Step 5); and a notification signal is output to the buzzer 50. By doing so, the buzzer 50 is activated, a notification sound is issued, and thus, the patient 7 or the like is notified that the capsule-type medical device 10 has passed through the stomach, that is, that the examination of the stomach has been completed (Step 6).

The patient 7 or a doctor collects the image information stored in the IC chip 32 by using the external device 60 on the basis of the notification from the capsule medical device 10. As shown in Figs. 5 and 6, by bringing the external device 60 close to the abdominal section of the patient 7, the capsule antenna 34 is activated by the image-reading signal issued from the external antenna 62, the image information stored in the IC chip 32 is read out, and the information is saved in the external memory 64 in the external device 60.

As has been described above, with the capsule-type medical device 10 according to this embodiment and the capsule-type medical system 1 including the same, when it is determined by the pH sensor 40 that the capsule-type medical device 10 has passed through the stomach in an examination of the stomach, the buzzer 50 issues the notification sound; therefore, the patient 7, the doctor, or the like who recognizes the notification sound can collect the image information of the stomach at an appropriate time before the examination of the stomach ends or before the capsule-type medical device 10 is excreted. Accordingly, the patient 7 does not have to continuously wear the external device 60 or to stay close to the external device 60; the burden on the patient 7, the feeling of being restrained in particular, can be considerably reduced; and the acquired image information can also be readily and reliably collected.

In the embodiment described above, the buzzer 50 has been described as an example of a notification unit; however, instead of this, for example, a vibrator 52 that vibrates when a notification signal is input from the sensor control unit 44 may be employed, as shown in Fig. 7. For example, by causing the vibrator 52 to vibrate when the capsule-type medical device 10 is about to be excreted, the patient 7 can be notified that the device is about to be excreted. By employing such a configuration, the patient 7 can be notified even in a noisy environment.

For example, a read-out restricting unit (not shown) that restricts transmission of the image information outside the body cavity until the passing position determined by the pH sensor 40 satisfies the predetermined positional condition may be employed. In this case, for example, when a determined passing position satisfies the predetermined positional condition, the sensor control unit 44 outputs a transmission-restriction-canceling signal to the read-out restricting unit and, by doing so, the read-out restriction unit activates the capsule antenna 34. By employing such a configuration, with the read-out restricting unit, it is possible to prevent image information with an insufficient amount of information from being accidentally read out before an examination of a desired site is completed, and, as a result, an incomplete diagnosis can be proactively prevented.

This embodiment can be modified as described below.
In this embodiment, the capsule-type medical device 10 is configured to observe returning light from a site to be examined; however, as a first modification, a capsule-type medical device 110 may be configured to observe fluorescence generated at a site to be examined. As shown in Fig. 8, the image-information acquisition unit 20 of the capsule-type medical device 110 according to this modification includes an LED 22 that emits broadband light, an excitation-light filter 123 that transmits only light of an excitation frequency (excitation light) in the light emitted from the LED 22, an excitation-light cut filter (fluorescence selecting unit) 125 that blocks the excitation light in the excitation light and the fluorescence from the site to be examined, and a photodetector (light detector) 124 that detects intensity information (measurement information) of the fluorescence that has passed through the excitation-light cut filter 125.

With the thus-configured capsule-type medical device 110, fluorescent substances existing in the site to be examined are excited by the excitation light emitted from the LED 22 and radiated onto the site to be examined in the body cavity, and the intensity information of the generated fluorescence is detected by the photodetector 124. The fluorescence generated from the fluorescent substances is fluorescence generated by a dye that is applied to the patient 7 or introduced thereinto in advance before the capsule-type medical device 110 is introduced into the patient 7 by means of intravenous injection, oral administration, etc. and that accumulates in the site to be examined, or autofluorescence generated from the site to be examined.

With the capsule-type medical device 110 according to the first modification, sensitivity and specificity for cancer are enhanced as compared with the case in which image information is acquired based on the returning light, and the diagnostic capability can be enhanced. In addition, as compared with the case in which the image information is acquired with the CCD 24, the amount of information stored in the IC chip 32 disposed in a limited space in the casing 12 can be reduced, and examination can readily be performed over an extended period of time.
Fluorescence image information (measurement information) may be acquired by employing a high-sensitivity CCD (fluorescence capturing unit) instead of the photodetector 124.

Although a case in which stomach examination is performed is described in this embodiment, without limitation to stomach examination, examination of other organs, for example, examination of the small intestine or large intestine, can also be performed in a similar manner. In addition, although the pH sensor unit 40 is described as an example of a passing-position determining unit, the passing position may be determined alternatively, or additionally, by the following modifications.
As shown in Fig. 9, a second modification may be configured such that, for example, a capsule-type medical device 210 includes an intestinal-bacteria sensor unit (passing-position determining unit) 240 that detects intestinal bacteria (positional information) instead of the pH sensor unit 40.

The intestinal-bacteria sensor unit 240 includes a pressure chamber 246 that is set at a predetermined pressure greater than atmospheric pressure and that has an opening 246a at a surface of the casing main body 12a, an azopolymer film 248 that is disposed so as to seal the opening 246a and that is broken by an enzyme secreted by the intestinal bacteria, and a pressure sensor 242 that detects the pressure inside the pressure chamber 246. The interior of the pressure chamber 246 is under a slight positive pressure.

With the thus-configured capsule-type medical device 210, the azopolymer film 248 is maintained so as not to be broken at sites to be examined other than the large intestine, and when the capsule-type medical device 210 moves to the large intestine, the azopolymer film 248 is ruptured by the enzyme secreted by the intestinal bacteria, causing the pressure in the pressure chamber 246 to decrease. Therefore, by detecting a drop in the pressure with the pressure sensor 242, it can be judged that the capsule-type medical device 210 has moved to the large intestine, and the patient 7 or the like can be notified that the examination of the small intestine has been completed.

The amount of time required for the capsule-type medical device 210 to reach the large intestine from the stomach varies among individuals. In addition, the retention time in the large intestine is generally 20 hours or longer, and it takes a long time for the capsule-type medical device 210 to be excreted after the small-intestine examination is completed.
With the capsule-type medical device 210 according to the second modification, the image information can be collected immediately after completion of the small-intestine examination, and the burden on the patient 7 can be reduced.

With this modification, a timer (passing-position determining unit, not shown) that measures elapsed time since the introduction into the body cavity may be employed along with the intestinal-bacteria sensor unit 240. As shown in Fig. 10, by employing the approximate elapsed time for reaching the large intestine after passing through the esophagus, the stomach, and the small intestine and the intestinal bacteria detected in the large intestine as positional information, passing positions can be determined more accurately. In Fig. 10, the longitudinal axis is the pressure value detected by the pressure sensor 242, and the horizontal axis is the elapsed time since the introduction of the capsule-type medical device 210 into the patient 7.
In this modification, the passage through the large intestine is determined by detecting the bacteria in the large intestine with the intestinal-bacteria sensor unit 240, and the timing of excretion is detected by measuring the time, with the timer, from the point at which it is determined that the capsule-type medical device 210 is passing through the large intestine.

As shown in Fig. 11, a third modification may be configured such that, for example, a capsule-type medical device 310 includes a pressure sensor unit (passing-position determining unit) 340 that detects the pressure (positional information) that the body-cavity wall surfaces exert on the casing 12, instead of the pH sensor unit 40.
The pressure sensor unit 340 includes the pressure chamber 246, the pressure sensor 242, a pressing member 346 that blocks the opening 246a, a spring-like elastic member 347 disposed between the pressing member 346 and the pressure sensor 242, and a cover 346 that covers the pressing member 346.

With the thus-configured capsule-type medical device 310, it can be judged that the device is in a relatively narrow organ, such as the esophagus, the small intestine, the large intestine, etc., when the pressure detected by the pressure sensor 242 is high, and that it is in a relatively large organ, such as the stomach, when the pressure is low. In addition, when the capsule-type medical device 310 is mixed with stool near the rectum, the pressure detected by the pressure sensor 242 via the pressing member 346 and the elastic member 347 increases. Therefore, if the pressure detected by the pressure sensor 242 exceeds a predetermined threshold, it can be judged by the sensor control unit 44 that the capsule-type medical device 310 is about to be excreted.

After a meal, food is generally retained for 2 hours in the stomach and 3 hours in the small intestine, and it reaches the entrance of the large intestine 5 hours after the meal. Then, the stool is excreted 24 hours to 48 hours after the meal. In addition, in an examination employing a capsule-type medical device, a meal should not be taken after 10PM on the previous day, and eating can be resumed about 4 hours after the examination is started. Assuming that the examination starts around 10AM, by the time the capsule-type medical device 310 reaches the rectum, it is mixed with stool from a meal taken before the examination, and, with further passage of time, the device is also mixed with stool from a meal taken after the examination. Because the area around the capsule-type medical device 310 is filled with stool at this time, the pressure that the pressure sensor 242 receives reaches a maximum. Therefore, at the sensor control unit 44, it can be judged, at the point where the preset pressure threshold is exceeded, that defecation is about to occur.
With the capsule-type medical device 310 according to the third modification, the image information can be reliably collected before excretion. Therefore, it is possible to avoid an accident in which the capsule-type medical device 310 is excreted and lost, and collection of the image information fails. In addition, it is possible to avoid the time-consuming and unsanitary procedure of collecting the image information after excretion.

As shown in Fig. 12, a fourth modification may be configured such that, for example, a capsule-type medical device 410 includes a timer (passing-position measuring unit) 443 in addition to the pH sensor 42. For example, as shown in Fig. 4, the pH changes to a slightly acidic value in the large intestine as compared with the small intestine. With the capsule-type medical device 410 according to this modification, in the case of performing a large-intestine examination, it can be judged that the capsule-type medical device 410 is passing through the large intestine at the point where 4 hours or more have passed since the device was introduced into the patient 7 and the pH value is 6.5 or lower. In addition, food generally reaches near the rectum about 13 hours after it reaches the large intestine and is excreted after 20 to 40 hours. Therefore, after detecting the movement of the capsule-type medical device 410 into the large intestine with the pH sensor 42, the buzzer 50 should be activated when the timer 443 measures that 20 hours have elapsed.

As a fifth modification, for example, a gas sensor (not shown) that detects gas (positional information) in the area around the casing 12 may be employed as a passing-position determining unit. The large intestine contains methane gas emitted from stool, and the concentration of the methane gas is especially high near the rectum where stool accumulates. Therefore, by detecting the gas in the area around the casing 12 with the gas sensor, it can be determined in a simple manner that the capsule-type medical device 10 has moved into the large intestine based on the presence of the methane gas. In addition, based on the amount of emitted methane gas, it can be judged whether or not the capsule-type medical device 10 is about to be excreted.

As a sixth modification, for example, a fluorescence detecting sensor (not shown) that detects fluorescence (measurement information) in the area around the casing 12 may be employed as a passing-position determining unit. With such a configuration, ICG (indocyanine green) is supplied to the body cavity in advance, stool dyed by being mixed with the ICG excreted from bile is detected with the fluorescence detecting sensor, and thus it is made possible to determine in a simple manner that the capsule-type medical device 10 has moved into the large intestine.

As a seventh modification, for example, a color detecting sensor (not shown) that detects color (positional information) in the area around the casing 12 may be employed as a passing-position determining unit. Hue is determined with the color detecting sensor from a two-dimensional image acquired by the image generating unit 26, and if a state in which the color of stool is being detected from the entire image continues for a certain amount of time or longer, it can be judged that the capsule-type medical device 10 is about to be excreted.

For example, an image processing unit (not shown) may be provided, and the distance moved to the large intestine may be detected by measuring the amount of movement by the capsule-type medical device 10 by processing images acquired by the image generating unit 26. In this case, for example, in combination with the intestinal-bacteria sensor unit 240, when it is judged by the intestinal-bacteria sensor unit 340 that the capsule-type medical device 10 has moved into the large intestine, the distance moved is calculated with the image processing unit, and it may be determined that the capsule-type medical device 10 is about to be excreted when the distance moved reaches about 1.5 m.

### {Second Embodiment}

Next, a capsule-type medical device according to a second embodiment of the present invention and a capsule-type medical system provided with the same will be described below.
As shown in Fig. 13, a capsule-type medical device 510 according to this embodiment differs from the one in the first embodiment in that a preliminary timer (passing-position determining unit) 570 that detects that a certain amount of time remains until completion of examination is additionally provided.
In the following, components that are common with the capsule-type medical devices 10, 110, 210, 310, and 410 and the capsule-type medical system 1 according to the first embodiment are given the same reference signs, and a description thereof will be omitted.

The preliminary timer 570 is configured to measure the amount of time that has passed since the capsule-type medical device 510 was introduced into the patient 7. In addition, the preliminary timer 570 outputs a preliminary notification signal to the buzzer 50 when a predetermined amount of time has passed, causing it to issue a preliminary notification sound. After food enters the stomach, it generally takes about 2 hours to move into the duodenum. For example, if the measuring time of the timer 570 is set at one hour, at a point where about one hour has passed since the capsule-type medical device 510 was introduced into the patient 7, the buzzer 50 is activated, and the preliminary notification sound is issued before the notification sound triggered by the pH sensor unit 40, which indicates the completion of examination.

By employing such a configuration, the patient 7 or the like who has recognized the preliminary notification sound determines that examination completion time is approaching and can prepare the external device 60 so that it is on hand, etc. Therefore, when the notification sound is issued once examination is completed, the image information can be collected quickly and at an appropriate timing. In addition, the risk of missing an appropriate timing for collecting the image information is reduced, and the burden of the examination on the patent 7 can be reduced.
A second buzzer may be provided as a preliminary notification unit separate from the buzzer 50 that serves as the notification unit.

As shown in Fig. 14, in this embodiment, the intestinal-bacteria sensor unit 240 may be employed instead of the pH sensor unit 40, and, separately from the notification sound issued immediately before excretion, a preliminary notification sound may be preliminarily issued outside the body cavity with the preliminary timer 570 a certain time before excretion. For example, it is judged that the capsule-type medical device 510 has moved into the large intestine at a point where the intestinal bacteria are detected by the intestinal-bacteria sensor unit 240, and the buzzer 50 is activated to issue the preliminary notification sound at this point. With this preliminary notification sound, the patient 7 can prepare the external device 60 so that it is on hand. Furthermore, the elapsed time is measured with the preliminary timer 570; when about 20 hours have passed since it was judged that the device has moved into the large intestine, it is determined that the device is about to be excreted; and the notification sound is issued again. The patient 7 can quickly collect the image information by using the external device 60 that has been prepared so that it is on hand.
The preliminary notification sound and the notification sound may be issued from separate notification units, or they may be distinguished by changing their tones or the way they sound. In addition, the vibrator 52 may be employed instead of the buzzer 50, and the buzzer 50 and the vibrator 52 may be employed in combination.

In this embodiment, for example, a gas sensor (passing-position determining unit) 572 that detects gas in the area around the casing 12 may be employed instead of the preliminary timer 570, as shown in Fig. 15. In this case, for example, the preliminary notification sound may be issued when the methane gas concentration reaches a certain threshold, and, furthermore, the notification sound immediately before excretion may be issued when a higher threshold is reached. Note that the preliminary notification sound may be issued multiple times by preparing a plurality of thresholds for issuing the preliminary notification sound. For example, the configuration may be such that the preliminary notification sound is issued once when the first threshold is reached, it is issued twice when the second threshold is reached, and, when the final threshold is reached, and excretion is about to occur, the notification sound is continuously issued. By doing so, the risk of accidentally missing the preliminary notification sound can be avoided, and the timing for preparing the external device 60 can be adjusted to an optimal timing.

### {Third Embodiment}

Next, a capsule-type medical device according to a third embodiment of the present invention and a capsule-type medical system provided with the same will be described.
As shown in Fig. 16, a capsule-type medical device 610 according to this embodiment differs from the one in the first embodiment in that the pH sensor 42, the intestinal-bacteria sensor unit 240, and a color detecting unit 674 that detects color (positional information) of the area around the casing 12 are provided as the passing-position measuring units and that the IC chip 32 stores the image information acquired by the CCD 24 in association with the positional information acquired by these passing-position determining units or the determined passing position information.
In the following, components that are common with the capsule-type medical devices 10, 110, 210, 310, 410, and 510 and the capsule-type medical system 1 according to the first embodiment or the second embodiment are given the same reference signs, and a description thereof will be omitted.

Because the pH value suddenly drops in the stomach, as shown in Fig. 17(a), the pH sensor 42 can precisely ascertain that the capsule-type medical device 610 has reached the stomach. In the same figure, the longitudinal axis is pH value and the horizontal axis is elapsed time. In addition, because the pressure suddenly drops in the large intestine, as shown in Fig. 17(b), the intestinal-bacteria sensor unit 240 can precisely ascertain that the capsule-type medical device 610 has reached the large intestine. In the same figure, the longitudinal axis is pressure value and the horizontal axis is elapsed time. Furthermore, color information of two-dimensional images acquired by the image generating unit 26 is analyzed at the color detecting unit 674, and it can be determined that the capsule-type medical device 610 is about to be excreted when a state in which the entire screen is filled with the color of stool continues for a certain amount of time or longer.

At the IC chip 32, the image information is stored in association with the positional information acquired by the pH sensor 42, the intestinal-bacteria sensor unit 240, and the color detecting unit 674 or the determined passing position information, thereby facilitating analyses of the image information.
As shown in Fig. 18, if the photodetector 124 is employed instead of the CCD 24 and only the fluorescence intensity information is saved in the IC chip 32, the information managing unit 30 can be reduced in size and can suitably be installed in the casing 12.

Although the embodiments of the present invention have been described in detail above with reference to the drawings, specific configurations are not limited to these embodiments, and design alterations, etc. within a range that does not depart from the spirit of the present invention are encompassed. For example, the present invention is not limited to the applications in the embodiments and modifications described above; the present invention may be applied in embodiments in which these embodiments and modifications are appropriately combined, and there is no particular limitation.
In addition, for example, the passing position may be determined by appropriately combining the pH sensor unit 40, the intestinal-bacteria sensor unit 240, the pressure sensor unit 340, the timer 443, the gas sensor 572, the fluorescence detecting sensor, and the color detecting sensor as the passing-position determining means. Also, the IC chip 32 may be configured to store the image information in association with the positional information or the determined passing position information.

### {Reference Signs List}

1 capsule-type medical system
10, 110, 210, 310, 410, 510, 610 capsule-type medical device
12 casing
20 image-information acquisition unit (measurement-information acquisition unit)
22 LED (light source)
32 IC chip (storage unit)
34 capsule antenna (transmitting/receiving unit)
40 pH sensor unit (passing-position determining unit)
50 buzzer (notification unit)
52 vibrator (notification unit)
60 external device (measurement-information collection device)
64 external memory (measurement-information saving unit)
124 photodetector (light detecting unit)
125 excitation-light cut filter (fluorescence selecting unit)
240 intestinal-bacteria sensor unit (passing-position determining unit)
340 pressure sensor unit (passing-position determining unit)
443 timer (passing-position determining unit)
572 gas sensor (passing-position determining unit)

## Claims

1. A capsule-type medical device comprising:
a capsule-type casing that is introduced into a body cavity of a patient;
a measurement-information acquisition unit that is disposed inside the casing and that acquires measurement information about an examination-target site in the body cavity;
a storage unit that stores the measurement information acquired by the measurement-information acquisition unit;
a transmitting/receiving unit that receives from outside the body cavity a measurement-information-reading signal and that transmits outside the body cavity the measurement information stored in the storage unit;
a passing-position determining unit that determines passing position by acquiring positional information in the body cavity ; and
a notification unit that reports the passing information outside the body cavity when the passing position determined by the passing-position determining unit satisfies a predetermined positional condition.

2. A capsule-type medical device according to Claim 1, wherein the measurement-information acquisition unit includes a light source that radiates excitation light onto the examination-target site, a fluorescence selecting unit that selectively transmits fluorescence of a predetermined band generated at the examination-target site by the excitation light irradiated from the light source, and a fluorescence capturing unit that acquires fluorescence image information by capturing the fluorescence that has passed through the fluorescence selecting unit.

3. A capsule-type medical device according to Claim 1, wherein the measurement-information acquisition unit includes a light source that radiates excitation light onto the examination-target site and a light detector that detects intensity information of fluorescence of a predetermined band generated at the examination-target site by the excitation light irradiated from the light source.

4. A capsule-type medical device according to Claim 1, wherein the storage unit stores the measurement information acquired by the measurement-information acquisition unit in association with the passing position determined by the passing-position determining unit.

5. A capsule-type medical device according to Claim 1, further comprising a read-out restricting unit that restricts the transmitting/receiving unit from transmitting the measurement information outside the body cavity until the passing position determined by the passing-position determining unit satisfies a predetermined positional condition.

6. A capsule-type medical device according to any one of Claims 1 to 5, wherein the passing-position determining unit is a timer that measures elapsed time.

7. A capsule-type medical device according to any one of Claims 1 to 5, wherein the passing-position determining unit is a pressure sensor that detects pressure exerted on the casing.

8. A capsule-type medical device according to any one of Claims 1 to 5, wherein the passing-position determining unit is an intestinal-bacteria sensor that detects intestinal bacteria.

9. A capsule-type medical device according to any one of Claims 1 to 5, wherein the passing-position determining unit is a pH sensor that detects a pH value in the area around the casing.

10. A capsule-type medical device according to any one of Claims 1 to 5, wherein the passing-position determining unit is a fluorescence detecting sensor that detects fluorescence in the area around the casing.

11. A capsule-type medical device according to any one of Claims 1 to 5, wherein the passing-position determining unit is a gas sensor that detects gas in the area around the casing.

12. A capsule-type medical device according to any one of Claims 1 to 5, wherein the passing-position determining unit is a color detecting sensor that detects color in the area around the casing.

13. A capsule-type medical system comprising:
a capsule-type medical device according to any one of Claims 1 to 12;
a measurement-information reading unit that transmits the measurement-information-reading signal and that reads the measurement information sent from the transmitting/receiving unit; and
a measurement-information saving unit that saves the read measurement information.
